# EUROPÄISCHE PATENTSCHRIFT

(11) **EP 2 460 107 B1**
(45) Veröffentlichungstag und Bekanntmachung des Hinweises auf die Patenterteilung: **10.10.2018**
(21) Anmeldenummer: 10737906.7
(22) Anmeldetag: 29.07.2010
(51) Int. Cl.: G06F 19/00

(54) **VERFAHREN ZUR ÜBERWACHUNG DES MEDIZINISCHEN ZUSTANDES EINES PATIENTEN**
METHOD FOR MONITORING THE MEDICAL STATE OF A PATIENT
PROCÉDÉ POUR CONTRÔLER L'ÉTAT MÉDICAL D'UN PATIENT

(30) Priorität: 29.07.2009 DE 102009035250
(43) Veröffentlichungstag der Anmeldung: 06.06.2012
(73) Patentinhaber: Meierhofer Medizintechnik GmbH, 04275 Leipzig (DE)
(72) Erfinder: LÖSER, Thomas, 04275 Leipzig (DE)
(74) Vertreter: Carlsohn, Alexander
(86) Internationale Anmeldenummer: PCT/EP2010/061036
(87) Internationale Veröffentlichungsnummer: WO 2011/012678

(56) Entgegenhaltungen:
- WO-A1-2006/136972
- WO-A2-01/93175
- US-A1- 2006 265 249
- US-A1- 2007 232 867
- PIWERNETZ K ET AL: "ANALYSIS AND PROCESSING OF DATA IN A HISPITAL-BASED DIABETES MANAGEMENT SYSTEM", HORMONE AND METABOLIC RESEARCH, THIEME-STRATTON, STUTTGART, DE, Bd. 24, Nr. SUPPL, 1. Januar 1990 (1990-01-01), Seiten 109-115, XP008067423, ISSN: 0018-5043

## Beschreibung

Die Erfindung betrifft ein Verfahren zur Überwachung des medizinischen Zustandes eines Patienten sowie ein System zur Ausführung dieses Verfahrens.

Im Gesundheitswesen, insbesondere in Kliniken, werden Patientendaten-Managementsysteme eingesetzt, in denen Patientendaten gespeichert werden. Die Patientendaten umfassen neben den sogenannten Stammdaten des Patienten wie Name, Vorname und Geburtsdatum die medizinischen Befunde und eingeleiteten oder bereits abgeschlossenen ärztlichen oder pflegerischen Maßnahmen. Derartige Maßnahmen werden im Folgenden auch als medizinische Maßnahmen bezeichnet.

Die medizinischen Befunde sind in der Regel Laborergebnisse, beispielsweise bei Herzerkrankungen relevante Messwerte wie der Myoglobin-Wert, der Troponin-I-Wert und der CKMB-Wert; bei respiratorischen Erkrankungen relevante Messwerte wie der Blutgasanalyse, z. B. der pH-Wert des Blutes, der Sauerstoff-Partikaldruck des Blutes, der Kohlendioxid-Partikaldruck des Blutes und die Sauerstoffsättigung des Blutes. Zu den medizinischen Befunden gehören ferner das Ergebnis der Untersuchung von Probenmaterial, das von dem Patienten gewonnen wurde, auf Infektionen durch pathogene Substanzen und/oder Organismen, beispielweise Keime wie Viren, Bakterien, Protisten, Pilze, Parasiten, Viroide oder Prionen.

Die Untersuchung des Patienten auf das Vorhandensein oder Fehlen einer Infektion mit pathogenen Substanzen und/oder Organismen ist vor allem bei der intensivmedizinischen Betreuung von Patienten von Bedeutung, insbesondere bei Patienten, die bereits an einer Sepsis leiden oder sich eine Sepsis während der Behandlung auf der Intensivstation zuziehen könnten.

Eine Sepsis ist definiert als "die Gesamtheit der lebensbedrohlichen klinischen Krankheitserscheinungen und pathophysiologischen Veränderungen als Reaktion auf die Aktion pathogener Keime und ihrer Produkte, die aus einem Infektionsherd in den Blutstrom eindringen, die großen biologischen Kaskadensysteme und spezielle Zellsysteme aktivieren und die Bildung und Freisetzung humoraler und zellulärer Mediatoren auslösen" (Schuster, H. P., und Müller-Werdan, U.: Definition und Diagnose von Sepsis und Multiorganversagen in Sepsis und MODS, Berlin 2005). Ist zusätzlich ein Organ des Patienten akut geschädigt, liegt eine schwere Sepsis vor.

Typischer Erreger einer Sepsis sind *Staphylococcus aureus,* koagulase-negative Staphylokokken, Enterkokken, *Escheria coli, Klebsiella spp., Enterbacter ssp.* und *Pseudomonas aeruginosa.*

Am Beginn einer intensivmedizinischen Behandlung, d. h. in der Regel bei der Einlieferung eines Patienten in die Intensivstation eines Krankenhauses, ist häufig nicht bekannt, ob der Patient an einer Infektion leidet oder nicht. Bekannt ist hingegen, welche Pathogene, insbesondere welche Keime, auf der Intensivstation bereits vorhanden sind. Aus diesem Grunde werden im Rahmen einer sogenannten "kalkulierten Therapie" sofort mit Beginn der intensivmedizinischen Behandlung ein oder mehrere Medikamente gegeben, mit denen typische Infektionen, insbesondere die Infektionen, die durch die Keime hervorgerufen werden, die auf der Station bekanntlich vorhanden sind, bekämpft werden sollen. Derartige Medikamente umfassen z. B. Antibiotika, Virostatika und Antimykotika, wobei die kalkulierte Therapie typischerweise die Gabe eines Antibiotikums vorsieht.

Gleichzeitig wird dem Patienten am Beginn der intensivmedizinischen Behandlung eine Probe entnommen, die auf pathogene Keime untersucht wird. Die Untersuchung, die beispielsweise mit dem Nachweis von Antikörpern oder der Erbinformation von Erregern (z. B. Specis-PCR) oder dem Anzüchten von Zellkulturen verbunden ist, wird in spezialisierten Labors durchgeführt. Die Ergebnisse dieser Untersuchung liegen typischerweise nach drei Tagen vor. Die Ergebnisse lassen erkennen, welche pathogenen Keime und welche Resistenzen bei dem Patienten vorliegen. Sobald dies bekannt ist, wird die medizinische Behandlung an den tatsächlichen Zustand des Patienten angepasst.

Die in den Patientendaten-Managementsystemen gespeicherten Daten werden durch die den Patienten behandelnden Personen sowie durch das Laborpersonal in das System über standardisierte Schnittstellen (in Krankenhäusern HL7, in Praxen xDT) eingegeben und auf Anforderung dem Behandler zur Verfügung gestellt.

Insbesondere bei multimorbiden Patienten ist die Anzahl an Daten, die für jeden einzelnen Patienten durch das Patientendaten-Managementsystem erfasst werden müssen, außerordentlich hoch. Aufgrund des erheblichen medizinischen Fortschrittes werden inzwischen jedoch bei vermeintlich einfachen Erkrankungen eine Vielzahl von Daten erfasst, die eine sachgerechte Analyse oft nicht zulassen. Es ist daher nicht untypisch, dass medizinische Entscheidungen anhand einzelner Daten, die als besonders auffallend gelten, getroffen werden. Andere, im Patientendaten-Managementsystem gespeicherte Daten, die einen anderen Befund stützen könnten, werden dabei außer acht gelassen, so dass aufgrund eher willkürlich ausgewählter Daten medizinische Maßnahmen getroffen werden, die sich bei vollständiger Analyse aller Daten als falsch oder unzureichend erweisen könnten.

Ein weiterer schwerwiegender Nachteil bekannter Patientendaten-Managementsysteme ist die erschwerte Kontrolle der Wirksamkeit der getroffenen medizinischen Maßnahmen, insbesondere der Medikation. Insbesondere ist bei bekannten Systemen nur schwer und in der Regel erst sehr spät feststellbar, welche Auswirkungen eine getroffene medizinische Maßnahme auf die Entwicklung des Gesundheitszustandes eines Patienten hat. Ferner ist es nicht untypisch, dass die Absetzung einer medizinischen Maßnahme, beispielsweise eines Medikamentes vergessen wird, und zwar deshalb, weil nicht auf den ersten Blick erkennbar ist, dass eine solche Verordnung existiert, welcher Zusammenhang zwischen dieser Verordnung und dem Zustand des Patienten existiert oder welchen Zweck die Verordnung ursprünglich haben sollte.

Überdies ist es ein häufiges Problem, dass tatsächlich nicht alle Daten des Patienten, die erfasst werden sollten, tatsächlich auch erfasst werden. Dennoch ist oft für die verantwortlichen Ärzte und Pfleger nicht zu erkennen, dass Daten fehlen.

Andererseits werden in der Praxis häufig Daten erfasst, die keinen Nutzen für die Diagnose und Therapie einer Erkrankung des Patienten haben. Beispielsweise werden nach starren Schemen Laborwerte angefordert, wobei je nach Wert beispielsweise tägliche oder wöchentliche Bestimmungen vorgenommen werden. Derartige Messungen sind zeit- und kostenaufwendig und können den Patienten zusätzlich belasten.

Aus der US 2007/287931 A1 ist ein System und ein Verfahren zur Überwachung des medizinischen Zustandes eines Patienten bekannt, das die Vorgabe einer Gruppe von physiologischen und/oder pathologischen Parametern des Patienten, die für das Vorliegen einer epileptischen Erkrankung charakteristisch sind, umfasst. Dabei werden die Parameter kontinuierlich bestimmt.

WO 2006/136972 offenbart ein Patientenüberwachungssystem mit einem Sensor, der physiologische Parameter in kurzen Intervallen erfasst, und einer Datenbank, in der Daten, die in längeren Intervallen erfasst werden, gespeichert werden. WO 01/93175 sieht die Erfassung von Daten vor, sobald diese Daten bei einem System eingehen, und nutzt diese Daten, um ärztliches Personal zu alarmieren, bevor ein kritischer Zustand des Patienten eintritt.

Aufgabe der Erfindung ist es, die Nachteile nach dem Stand der Technik zu beseitigen. Es soll insbesondere ein Verfahren zum Überwachen des medizinischen Zustandes eines Patienten angegeben werden, dass eine Erfolgskontrolle getroffener medizinischer Maßnahmen und damit eine sachgerechte medizinische Behandlung ermöglicht, womit eine deutliche Reduzierung der Behandlungszeit eines Patienten verbunden sein kann. Überdies soll sichergestellt werden, dass tatsächlich sämtliche Daten eines Patienten, die gemäß der getroffenen Vorgaben erfasst werden sollen, auch tatsächlich erfasst werden, wobei die Vorgaben so getroffen werden sollen, dass bestimmte Daten nur dann erfasst werden, wenn sie medizinisch notwendig ist. Ferner soll ein System zur Ausführung dieses Verfahrens angegeben werden.

Diese Aufgabe wird durch die Merkmale von Anspruch 1 gelöst. Zweckmäßige Ausgestaltungen der Erfindungen ergeben sich aus den Merkmalen der Unteransprüche.

Nach Maßgabe der Erfindung ist ein Verfahren zur Überwachung des medizinischen Zustandes eines Patienten mittels eines computerimplementierten Systems, das einen Prozessor, einen Speicher, eine Eingabevorrichtung und eine Anzeigevorrichtung aufweist, vorgesehen, umfassend
(a) die Vorgabe, durch einen Arzt mittels der Eingabevorrichtung, einer ersten Gruppe von physiologischen und/oder pathologischen Parametern des Patienten, die für das Vorliegen einer Gesundheitsstörung charakteristisch sind, wobei die Parameter der ersten Gruppe kontinuierlich bestimmt werden sollen, und die Vorgabe einer zweiten Gruppe von physiologischen und/oder pathologischen Parametern des Patienten, die für das Vorliegen der Gesundheitsstörung charakteristisch sind, wobei die Parameter der zweiten Gruppe diskontinuierlich bestimmt werden sollen;
(b) das kontinuierliche Erfassen von Werten von Parametern der ersten Gruppe, wobei die kontinuierlich erfassten Werte in dem Speicher gespeichert werden;
(c) das kontinuierliche Bestimmen der Notwendigkeit, Werte von Parametern der zweiten Gruppe zu bestimmen, auf Basis von zuvor erfassten Werten von Parametern der ersten Gruppe oder auf Basis von zuvor erfassten Werten von Parametern der ersten Gruppe und der zweiten Gruppe mittels des Prozessors; und
(d) das Erfassen von Werten von Parametern der zweiten Gruppe, wenn in Schritt (c) die Notwendigkeit der Erfassung von Werten von Parametern der zweiten Gruppe festgestellt wurde, zu jedem Zeitpunkt, an dem eine solche Notwendigkeit festgestellt wurde, wobei die Werte in dem Speicher gespeichert werden;
wobei
- ein Parameter kontinuierlich erfasst wird, wenn er ununterbrochen oder in Abständen von 24 h oder weniger bestimmt wird, und ein Parameter diskontinuierlich erfasst wird, wenn er in regelmäßigen oder unregelmäßigen Abständen von mehr als 24 h oder nur dann bestimmt wird, wenn sich dies in Schritt (c) ergibt;
- zur Bestimmung der Notwendigkeit, Parameter der zweiten Gruppe zu bestimmen (Schritt (c)), für zwei oder mehr Parameter der ersten Gruppe jeweils Auslösewerte oder Auslösewertebereiche vorgegebenen sind, bei deren Erreichen zumindest ein Wert zumindest eines Parameters der zweiten Gruppe bestimmt werden muss; und
- die zeitlichen Abstände zwischen zwei aufeinanderfolgenden Bestimmungen von Parametern, die der zweiten Gruppe zugeordnet sind, erfasst werden und dass ein Parameter aus der zweiten Gruppe in die erste Gruppe überführt wird, wenn eine vorgegebene Anzahl von aufeinanderfolgenden zeitlichen Abständen, die geringer als eine vorgegebene Zeiteinheit sind, erreicht wird, wobei die vorgegebene Anzahl eine Ganzzahl größer gleich zwei ist und die vorgegebene Zeiteinheit 24 Stunden oder weniger ist;
wobei die Bestimmung der Notwendigkeit, Parameter der zweiten Gruppe zu bestimmen (Schritt (c))
(c1) das Auswählen von zumindest drei Parametern aus der ersten oder zweiten Gruppe, wobei zumindest einer der drei Parameter ein Parameter der ersten Gruppe ist,
(c2) das Bilden eines mehrdimensionalen Merkmalsraums, wobei jeder der ausgewählten Parameter eine Dimension des Merkmalsraums bildet und die Zeit eine weitere Dimension des Merkmalsraums bildet;
(c3) das Bestimmen von zumindest einem ersten Raumbereich und von zumindest einem zweiten Raumbereich innerhalb des Merkmalsraums, wobei
   im ersten Raumbereich jeder der ausgewählten Parameter und/oder die Zeit Werte aufweist, die als Zielwerte vorgegeben worden sind;
   im zweiten Raumbereich zumindest einer der Parameter und/oder die Zeit Werte aufweist, die als unerwünschte Werte vorgegeben worden sind;
(c4) das kontinuierliche Einordnen der erfassten Werte für die in Schritt (c1) ausgewählten Parameter, in den Merkmalsraum, wodurch Messpunkte in dem Merkmalsraum gebildet werden; und
(c5) das Feststellen der Notwendigkeit, Parameter der zweiten Gruppe zu erfassen, sobald ein Messpunkt im zweiten Raumbereich liegt;
umfasst.

Das erfindungsgemäße Verfahren bietet den Vorteil, dass Werte für die Parameter der zweiten Gruppe nur dann erfasst werden, wenn sich aus den bisher ermittelten Parametern die Notwendigkeit der Bestimmung dieser Parameter ergibt. Damit wird verhindert, dass nach starren Schemen Werte für Parameter bestimmt werden, die für die Diagnose und Therapie der Gesundheitsstörung nicht benötigt werden. Statt dessen werden Werte dieser Parameter nur dann bestimmt, wenn sich aus den bisher bestimmten Parametern ergibt, dass diese Werte benötigt werden.

Die zweite Gruppe der Parameter umfasst zweckmäßigerweise solche Parameter, die in der bisherigen medizinischen Praxis in Abständen von 24 Stunden oder mehr bestimmt werden, beispielsweise mikrobiologische Parameter und/oder labormedizinische Parameter, insbesondere solche Parameter, die für das Vorliegen einer Infektion charakteristisch sind. Die zweite Gruppe von Parametern umfasst beispielsweise Parameter, die mittels Blutanalysen gewonnen werden, und/oder Ergebnisse bakteriologischer, virologischer und mykologischer Untersuchungen oder Untersuchungen auf andere Keime wie beispielsweise Prionen.

Gemäß der Erfindung werden Untersuchungen auf pathogene Keime somit nur dann durchgeführt, wenn sich aus den bereits vorhandenen Daten ergibt, dass die Untersuchungsergebnisse für die Diagnose und/oder Therapie von Nutzen sein könnten. Ebenso wenig ist es noch erforderlich, täglich Laborwerte zu erfassen. Statt dessen wird aus den vorhandenen Werten der erfassten Parameter ermittelt, ob bestimmte Laborwerte benötigt werden.

Erfindungsgemäß ist vorgesehen, dass kontinuierlich anhand der vorhandenen Daten, d. h. der bisher erfassten Werte von Parametern der ersten und - falls bereits vorhanden - der zweiten Gruppe von Parametern, überprüft wird, ob Werte für die Parameter der zweiten Gruppe zu erfassen sind. Die Notwendigkeit einer solchen Erfassung kann beispielsweise festgestellt werden, indem für zumindest einen Parameter der ersten Gruppe ein Auslösewert oder ein Auslösewertebereich vorgegeben ist. Erreicht der kontinuierlich erfasste Wert dieses vorgegebenen Parameters den Auslösewert oder den Auslösewertebereich, so ist es notwendig, zumindest einen Wert zumindest eines Parameters der zweiten Gruppe zu bestimmen. Dabei ist vorgegeben, für welchen Parameter oder für welche Parameter der zweiten Gruppe Werte zu bestimmten sind, wenn der Auslösewert oder ein Auslösewertebereich des vorgegebenen ersten Parameters erreicht wird. Dabei können verschiedene erste Parameter mit dem- oder denselben Parametern der zweiten Gruppe verknüpft sein, d. h. erreicht nur einer der kontinuierlich erfassten Werte von Parametern der ersten Gruppe einen Auslösewert oder den Auslösewertebereich, so steht die Notwendigkeit der Erfassung eines Wertes eines Parameters oder von Werten unterschiedlicher Parameter der zweiten Gruppe fest. Ferner sind Parameter der ersten Gruppe so miteinander verknüpft, das Auslösewerte oder Auslösewertebereiche für zwei oder mehr dieser Parameter erreicht werden müssen, bevor die Notwendigkeit feststeht, Werte für Parameter der zweiten Gruppe bestimmen zu müssen.

Die Notwendigkeit, Parameter der zweiten Gruppe zu bestimmen, wird mittels folgenden Verfahrens vorgenommen:
(c1) das Auswählen von zumindest drei Parametern aus der ersten oder zweiten Gruppe, wobei zumindest einer der drei Parameter ein Parameter der ersten Gruppe ist,
(c2) das Bilden eines mehrdimensionalen Merkmalsraums, wobei jeder der ausgewählten Parameter eine Dimension des Merkmalsraums bildet und die Zeit eine weitere Dimension des Merkmalsraums bildet;
(c3) das Bestimmen von zumindest einem ersten Raumbereich und von zumindest einem zweiten Raumbereich innerhalb des Merkmalsraums, wobei
   im ersten Raumbereich jeder der ausgewählten Parameter und/oder die Zeit Werte aufweist, die als Zielwerte vorgegeben worden sind;
   im zweiten Raumbereich zumindest einer der Parameter und/oder die Zeit Werte aufweist, die als unerwünschte Werte vorgegeben worden sind;
(c4) das kontinuierliche Einordnen der erfassten Werte für die in Schritt (c1) ausgewählten Parameter, in den Merkmalsraum, wodurch Messpunkte in dem Merkmalsraum gebildet werden; und
(c5) das Feststellen der Notwendigkeit, Parameter der zweiten Gruppe zu erfassen, sobald ein Messpunkt im zweiten Raumbereich liegt.

Liegt ein Messpunkt im zweiten Raumbereich, so werden Werte für einen vorgegebenen Parameter der zweiten Gruppe oder für mehrere vorgegebene Parameter der zweiten Gruppe bestimmt, je nach dem, welche Vorgaben für den Fall getroffen wurden, dass ein Messpunkt in einem zweiten Raumbereich liegt. Nach dem Erfassen der Werte der Parameter der zweiten Gruppe kann ein neuer zweiter Raumbereich definiert werden, der die bisher erfassten Werte der ersten und der zweiten Gruppe berücksichtigt. Ferner können bei der Definition des zweiten Raumbereiches auch die bisher getroffenen medizinischen Maßnahmen, beispielsweise die Gabe eines Medikamentes berücksichtigt werden.

Auf die ermittelte Notwendigkeit zur Bestimmung von Werten eines oder mehrerer Parameter der zweiten Gruppe kann mittels eines akustischen oder optischen Alarms auf einer Anzeigevorrichtung, beispielsweise dem Display eines Client-Computers des Patientendaten-Managementsystems hingewiesen werden.

Bei der Ausführung des erfindungsgemäßen Verfahrens kann sich in Schritt (c) herausstellen, dass ein Parameter, der ursprünglich der zweiten Gruppe zugeordnet worden ist, kontinuierlich bestimmt werden muss. Dieser Parameter kann dann von der zweiten in die erste Gruppe überführt werden. Beispielsweise kann vorgesehen sein, dass ein Parameter der zweiten Gruppe automatisch in die erste Gruppe überführt wird, wenn in Schritt (c) festgestellt wird, das Bestimmungen dieses Parameters in zwei oder mehr aufeinanderfolgenden Abständen von weniger als 24 Stunden notwendig war.

Das erfindungsgemäße Verfahren kann ferner einen oder mehrere der folgenden Schritte umfassen:
(e) das Erfassen der getroffenen medizinischen Maßnahmen für den Patienten, wobei für jede getroffene medizinische Maßnahme zumindest der zeitliche Beginn der medizinischen Maßnahme erfasst wird und die medizinische Maßnahme zumindest einem der erfassten Parameter der ersten Gruppe, der mit der medizinischen Maßnahme beeinflusst werden soll, zugeordnet wird;
(f) das Zuordnen der getroffenen medizinischen Maßnahme zu einer vorgegebenen Kategorie, wobei jede Kategorie eine Vielzahl von potentiellen medizinischen Maßnahmen umfasst;
(g) das Zuordnen der Kategorie, der die medizinische Maßnahme in Schritt (c) zugeordnet worden ist, zu einem der erfassten Parameter der ersten Gruppe; und
(h) die gemeinsame und zeitabhängige Darstellung (i) der Kategorie, (ii) des in Schritt (g) zugeordneten Parameters der ersten Gruppe und (iii) zumindest eines Parameters der zweiten Gruppe.

Die gemeinsame und zeitabhängige Darstellung (i) der Kategorie, (ii) des zugeordneten Parameters der ersten Gruppe und (iii) zumindest eines Parameters der zweiten Gruppe ermöglicht es einem Behandler, sofort zu erkennen, welcher Zusammenhang zwischen dem Zustand des Patienten, für diesen Zustand charakteristischen Werten von Parametern und den getroffenen medizinischen Maßnahmen besteht. Beispielsweise kann der Behandler sofort erkennen, ob der Patient an einer Infektion leidet, die eine Sepsis hervorgerufen hat oder hervorrufen wird. In diesem Fall ist der zweite Parameter ein mikrobiologischer Parameter. Vorzugsweise umfasst die gemeinsame und zeitabhängige Darstellung in Schritt (h) die Darstellung von zumindest zwei Parametern der zweiten Gruppe, wobei einer der beiden Parameter ein mikrobiologischer Parameter und der andere der beiden Parameter ein labormedizinischer Parameter ist.

Die Zuordnung erfolgt in den Schritten (e) und (d) jeweils zu einem Parameter der ersten Gruppe, da nur diese Parameter kontinuierlich erfasst werden. Unter einem Parameter der ersten Gruppe kann auch ein Parameter verstanden werden, der ursprünglich der zweiten Gruppe zugeordnet gewesen ist, der dann aber von der zweiten Gruppe in die erste Gruppe überführt wurde, weil in Schritt (c) festgestellt wurde, dass der Parameter kontinuierlich bestimmt werden muss.

In einer Ausführungsform der Erfindung kann vorgesehen sein, dass ein optischer oder/und akustischer Alarm ausgelöst wird, wenn bei der Erfassung von Werten von Parametern der zweiten Gruppe ein vorgegebener pathogener Keim und/oder eine Resistenz eines festgestellten Keims gegen ein Arzneimittel festgestellt wird, das dem Patienten verordnet werden soll.

Vorzugsweise ist die getroffene medizinische Maßnahme die Verabreichung eines Arzneimittels, insbesondere die Verabreichung eines Medikamentes.

Die Kategorien werden bevorzugt auf der Basis vorgegebener Merkmale gebildet, die jeweils nur einem Teil der potentiellen medizinischen Maßnahmen zueigen sind. Solche Merkmale sind beispielsweise der Wirkstoff des Arzneimittels, die Wirkstoffgruppe dieses Arzneimittels und/oder die beabsichtigte pharmakologische Wirkung dieses Arzneimittels.

Für jede medizinische Maßnahme, die für einen bestimmten Patienten getroffen werden soll, wird der Beginn der medizinische Maßnahme erfasst. Zweckmäßigerweise werden jedoch auch weitere Angaben zu dieser medizinischen Maßnahme in Schritt (e) erfasst. Derartige weitere Angaben können aus der Gruppe ausgewählt sein, die beispielsweise das vorgesehene Ende der getroffenen medizinischen Maßnahme, das tatsächliche Ende der getroffenen medizinischen Maßnahme, den Namen des verordneten Arzneimittels, die pharmakologischen Parameter wie die Darreichungsform, die Dosis, die Häufigkeit der Dosis pro Tag und die zeitliche Verteilung der Dosierung über den Tag und die Veränderung der Dosierung über einen vorgegebenen Zeitraum sowie Kombinationen dieser Angaben umfassen.

In einer bevorzugten Ausführungsform wird der Name des verordneten Arzneimittels erfasst, wobei anhand des Namens des vorordneten Arzneimittels die Kategorie der getroffenen medizinischen Maßnahme unter Rückgriff auf eine Datenbank bestimmt wird, in der der Name des Arzneimittels mit zumindest einem der Merkmale Wirkstoff, Wirkstoffgruppe oder beabsichtigte pharmakologische Wirkung verknüpft ist. Beispielsweise kann die Angabe des Namens "Nebilet" oder des Namens "Lobivon" mit dem Wirkstoff "Nebivolol", der Wirkstoffgruppe "β1-Adrenorezeptorenblocker" und der pharmakologischen Wirkung "Senkung von Pulsfrequenz und Blutdruck" verknüpft sein.

Da es für den Erfolg einer medizinischen Maßnahme in der Regel ohne Interesse ist, wie das Arzneimittel benannt ist, sondern nur dessen Wirkstoff oder Wirkstoffgruppe für einen solchen Erfolg von Belang ist, kann die Bildung der Kategorie beispielsweise nach dem Merkmal "Wirkstoff vorgenommen werden. D. h in Schritt (h) würde neben dem zugeordneten Parameter der ersten Gruppe beispielsweise die Kategorie "β1-Adrenorezeptorenblocker", die auf Basis des Merkmals "Wirkstoffgruppe" gebildet worden ist, angezeigt werden. Der Behandler erkennt in Schritt (h) sofort, dass ein β1-Adrenorezeptorenblocker verordnet worden ist; welcher β1-Adrenorezeptorenblocker dies im konkreten tatsächlich ist, ist dabei ohne Belang. Selbstverständlich kann der Behandler, sofern er dies für erforderlich erachtet, Einzelheiten zu den verordneten medizinischen Maßnahmen aufrufen, die in Schritt (e) erfasst worden sind.

Die Zuordnung einer medizinischen Maßnahme zu einer Kategorie in Schritt (e) wird vorzugsweise automatisch ausgeführt, d. h., dass der Behandler die Zuordnung der von ihm getroffenen Maßnahme nicht selbst vornehmen muss. Vielmehr wird anhand der getroffenen Maßnahme automatisch bestimmt, welcher Kategorie diese Maßnahme zugeordnet ist.

In einer Ausführungsform der Erfindung ist der Parameter der ersten Gruppe, dem die Kategorie in Schritt (g) zugeordnet wird, derselbe Parameter wie der Parameter der ersten Gruppe, dem die medizinische Maßnahme in Schritt (e) zugeordnet worden ist. Alternativ oder zusätzlich ist der Parameter, dem die Kategorie in Schritt (g) zugeordnet wird, ein anderer Parameter als der Parameter, dem die medizinische Maßnahme in Schritt (e) zugeordnet worden ist. Letztere Ausführungsform kann insbesondere im Hinblick auf das breite Wirkungsspektrum zahlreicher Arzneimittel von Vorteil sein. Verordnet ein Behandler beispielsweise einen β1-Adrenorezeptorenblocker, um eine Senkung des Blutdruckes zu erreichen, so kann dies Auswirkungen auf die Leberfunktion des Patienten haben. In diesem Fall kann es empfehlenswert sein, die Kategorie "β1-Adrenorezeptorenblocker" nicht nur mit den Messwerten des Blutdruckes (das ist ein Parameter der ersten Gruppe) zu verknüpfen, sondern auch mit Parametern der ersten Gruppe, die im Zusammenhang mit der Leberfunktion des Patienten stehen, beispielsweise mit Messwerten der Glutamat-Pyruvat-Transaminase (GPT), sofern dieser Wert kontinuierlich bestimmt werden muss.

Der oder die Parameter der zweiten Gruppe, die gemäß Schritt (h) dargestellt werden, können vom Behandler vorgegeben werden oder werden automatisch bestimmt, indem bestimmte zweite Parameter bestimmten ersten Parametern zugeordnet werden, d. h. der oder die angezeigten zweiten Parameter können mit den in Schritt (e) und/oder (g) ausgewählten Parametern der ersten Gruppe verknüpft sein, so dass die Zuordnung eines Parameters der ersten Gruppe in Schritt (e) und/oder (g) den oder die Parameter der zweiten Gruppe vorgibt, der in Schritt (h) angezeigt werden soll.

Die in Schritt (h) angezeigten Parameter der ersten und zweiten Gruppe werden im Folgenden gemeinsam auch als ausgewählte Parameter bezeichnet.

Vorzugsweise werden in Schritt (h) die Kategorie und die ausgewählten Parameter der ersten und zweiten Gruppe in einem Diagramm dargestellt, dessen Abszisse die Zeit bildet, wobei in dem Diagramm Messwerte der ausgewählten Parameter und die Kategorie der getroffenen Maßnahme dargestellt werden. Dabei wird die Kategorie zweckmäßigerweise als Balken, der parallel zur Abszisse verläuft, in dem Diagramm gezeigt, wobei insbesondere der Beginn der Maßnahme als Balkenanfang in dem Diagramm dargestellt ist. Mit dem Ende der Maßnahme endet die Darstellung der Kategorie, beispielsweise des Balkens, in dem Diagramm. In einer weiteren Ausführungsform der Erfindung ist die Kategorie der getroffenen Maßnahme als farbig kodierter Balken dargestellt, wobei die farbige Kodierung mit dem vorgegebenen Merkmal verknüpft ist.

Unter dem Begriff "physiologischer und/oder pathologischer Parameter" ist ein Wert zu verstehen, der (a) den Messwert einer gemessenen physiologischen Kenngröße eines Patienten oder eines Indikatorstoffes angibt (z.B. den Sauerstoffpartialdruck bei einer Blutgasanalyse, Atemfrequenz, Körpertemperatur, CKMB-Konzentration; Keimbelastung); und (b) eine Klassifizierung eines physiologischen Zustandes eines Patienten (z. B. gelbe Färbung der Gesichtshaut; Vorhandensein eines bestimmten Keims; Bestehen einer Resistenz) umfassen kann.

Ein Parameter wird der ersten Gruppe zugeordnet, wenn er kontinuierlich erfasst wird. Unter kontinuierlichem Erfassen wird das ununterbrochene Erfassen von Werten des Parameters oder das Erfassen von Werten des Parameters in Abständen von 24 h oder weniger verstanden.

Ein Parameter wird der zweiten Gruppe zugeordnet, wenn er diskontinuierlich erfasst wird. Unter diskontinuierlichem Erfassen wird das einmalige Erfassen von Werten des Parameters; das Erfassen von Werten des Parameters in regelmäßigen oder unregelmäßigen Abständen von mehr als 24 Stunden; oder das vollkommene Fehlen der Erfassung von Werten für diesen Parameter verstanden, wenn sich in Schritt (c) zu keinem Zeitpunkt des erfindungsgemäßen Verfahrens ergibt, dass die Erfassung von Werten für diesen Parameter notwendig ist.

Ergibt sich in Schritt (c) des erfindungsgemäßen Verfahrens, dass ein Wert, der ursprünglich der zweiten Gruppe zugeordnet worden ist, in Abständen von 24 Stunden oder weniger erfasst werden muss, so wird dieser Parameter von der zweiten Gruppe in die erste Gruppe eingeordnet. Eine solche Notwendigkeit kann beispielsweise dann vorliegen, wenn ein Parameter, der ursprünglich der zweiten Gruppe zugeordnet worden ist, in zwei oder mehr aufeinander folgenden Abständen von weniger als 24 Stunden bestimmt worden ist. Dazu kann in dem erfindungsgemäßen Verfahren vorgesehen sein, die Abstände zwischen zwei aufeinanderfolgenden Bestimmungen der Parameters, die der zweiten Gruppe zugeordnet sind, zu erfassen und zu überprüfen, ob zwei oder mehr aufeinanderfolgende Abstände zwischen Erfassungen desselben Parameters 24 Stunden oder weniger betragen. Die Anzahl der hierfür erforderlichen aufeinanderfolgenden Abstände, die 24 Stunden oder weniger betragen, sollte eine Ganzzahl größer oder gleich 2 sein.

Wird ein Parameter von der zweiten Gruppe in die erste Gruppe überführt, so kann vorgesehen sein, dass eine Alarm ausgelöst wird und/oder dass die Kategorie in Schritt (e) diesem Parameter, der nun ein Parameter der ersten Gruppe ist, zugeordnet wird.

Der Ausdruck "Indikatorstoff' bezieht sich hierin auf Verbindungen oder Elemente, die - je nach ihrer Art - in biologischen Systemen produziert werden oder in biologische Systeme eingebracht werden und deren Vorhandensein oder deren Konzentration (z. B. in einem bestimmten Organ) ein Charakteristikum für einen biologischen Prozess oder einen biologischen Zustand ist. Derartige Verbindungen und Elemente umfassen beispielsweise solche, die von Tumorzellen produziert, durch einen Tumor in anderen Körperzellen induziert und/oder als tumorspezifische Stoffe in ihrer Konzentration durch einen Tumor verändert werden. Derartige Indikatorstoffe sind beispielsweise Makromoleküle, z. B. Proteine, oder Spurenelemente. Derartige Verbindungen und Elemente umfassen weiterhin Bonemarker, die für Knochenabbauprozesse wie Osteoporose charakteristisch sind oder Enzyme, die für die Beurteilung der Funktion von Organen wichtig sind.

Der Begriff "Wert" oder "Messwert" bezieht sich hier auf alle im medizinischen Bereich (a) anfallende Zahlenwerte für einen Parameter (z. B. einen Sauerstoffpartialdruck bei einer Blutgasanalyse in Höhe von 81,2 mmHg, eine Atemfrequenz von 15 Atemzügen pro Minute, eine Körpertemperatur von 36,8 °C, CKMB-Konzentration von 152 ng/ml; Keimbelastung ...) oder (b) auf Klassifizierungsergebnisse eines physiologischen Zustandes eines Patienten (z. B. gelbe Färbung der Gesichtshaut: nein, wobei Klassifizierungen mittels Aussagen wie "Nein" ein Zahlenwert, beispielsweise "0" zugeordnet werden sollte).

Vorzugsweise werden zumindest die Werte eines der physiologischen Parameter, die für das Vorliegen einer Gesundheitsstörung charakteristisch sind, unter Verwendung eines Indikatorstoffes bestimmt.

Dabei sollten physiologische Parameter ausgewählt werden, von denen aufgrund medizinischer Erkenntnisse angenommen wird, dass sie im Zusammenhang mit einer bestimmten Gesundheitsstörung, beispielsweise einer respiratorischen Insuffizienz, stehen. Zur Bestimmung des medizinischen Risikos einer respiratorischen Insuffizienz werden zum Beispiel physiologische Parameter eingesetzt, die mittels einer Blutgasanalyse gewonnen werden. Die mittels der Blutgasanalyse gewonnenen physiologischen Parameter können den pH-Wert des Blutes, den Sauerstoff-Partialdruck des Blutes, den Kohlendioxid-Partialdruck des Blutes und die Sauerstoffsättigung des Blutes umfassen. Weitere physiologische Parameter, die neben den aus der Blutgasanalyse stammenden Werten zur Abschätzung des medizinischen Risikos einer respiratorischen Insuffizienz in dem erfindungsgemäßen Verfahren verwendet können, umfassen die Atemfrequenz (AF) des Patienten sowie das Alter und das Geschlecht des Patienten.

Die Zahl von Parametern der ersten Gruppe, deren Werte in Schritt (a) erfasst werden, sollte mindestens 2 betragen. Die Zahl von Parametern der zweiten Gruppe, deren Werte in Schritt (d) erfasst werden, sollte mindestens 1, vorzugsweise mindestens 2 betragen.

Der Begriff "medizinische Maßnahme" umfasst jede ärztliche oder pflegerische Maßnahme, die unternommen wird, um den Gesundheitszustand des Patienten zu verbessern, beispielsweise einen oder mehrere der erfassten physiologischen Parameter in einen Bereich zu führen, der bei gesunden Personen typischerweise vorliegt.

Die getroffenen medizinischen Maßnahmen können ebenfalls kontinuierlich erfasst werden, d. h. jede weitere Maßnahme, jede Veränderung einer bestehenden Maßnahme (z. B. der Dosierung eines Medikamentes) und/oder das tatsächliche Ende einer Maßnahme werden erfasst.

In einer weiteren Ausführungsform des erfindungsgemäßen Verfahrens kann ein zusätzlicher Schritt vorgesehen sein, bei dem die Vollständigkeit der Daten überprüft wird. Die Überprüfung der Vollständigkeit der Daten kann kontinuierlich erfolgen. Alternativ wird sie jeweils nach Ablauf einer vorgegebenen Zeitspanne, beispielsweise aller vier, acht, sechzehn und/oder vierundzwanzig Stunden, durchgeführt. Diese Zeitspannen können der Schichtdauer oder einem Arbeitstag des Krankenhauses entsprechen.

Die Daten werden dann als vollständig angesehen, wenn sie den medizinischen Vorgaben, insbesondere den getroffenen medizinischen Maßnahmen sowie den physiologischen Parametern und sonstigen Informationen, die gemäß den Vorgaben des Krankenhauses im allgemeinen und den Vorgaben des Arztes im besonderen zu erfassen sind, entsprechen.

Die Daten, die gemäß den medizinischen Vorgaben zu erfassen sind, werden im folgenden auch als "erwartete Daten" bezeichnet, da deren Erfassung gemäß den medizinischen Vorgaben erwartet wird. Einzelne erwartete Daten werden als "erwartete Angabe" bezeichnet.

Um die Vollständigkeit der Daten zu überprüfen, werden die tatsächlich erfassten Daten mit den Daten verglichen, die gemäß den medizinischen Vorgaben zu erfassen sind. Die erfassten Daten werden dabei den erwarteten Daten zugeordnet. Wird beispielsweise die Erfassung des Sauerstoffpartialdruckes erwartet, so wird der tatsächlich gemessene und erfasste Wert des Sauerstoffpartialdruckes dem erwarteten Wert zugeordnet. Jede erwartete Angabe kann mit einer oder mehreren Zeitangaben verknüpft sein, aus der sich ergibt, wann ein Wert für die erwartete Angabe erwartet wird.

Der Vergleich der erfassten Daten mit den erwarteten Daten ergibt entweder, dass (i) allen erwarteten Daten auch erfasste Daten entsprechen, d.h., dass alle erwarteten Daten auch erfasst worden sind; dass (ii) nur einem Teil der erwarteten Daten auch erfassten Daten entsprechen, d.h., dass nur ein Teil der erwarteten Daten auch erfasst worden ist, während ein anderer Teil der erwarteten Daten nicht erfasst worden ist; oder dass (iii) keinen der erwarteten Daten auch erfasste Daten entsprechen, d.h., dass keine der erwarteten Daten auch erfasst worden sind. In den Fällen (ii) und (iii) wird vorzugsweise eine Warnung nach einem vorgegebenen Zeitintervall ausgegeben. Dazu können die Felder in der Bildschirmdarstellung beispielsweise mit einer Farbkodierung versehen sein. Vorzugsweise wird die Farbe der Feldumrandung und/oder der Feldbeschriftung verändert, wenn einer der Fälle (ii) oder (iii) eintritt. In einer bevorzugten Ausführungsform wird eine Warnung nur zu den vorgegebenen Zeitspannen ausgegeben, also aller vier, acht, sechzehn und/oder vierundzwanzig Stunden, um eine Dauerwarnung, jeweils für eine andere erwartete Angabe, zu vermeiden. Mit anderen Worten, ist eine Überprüfung der Vollständigkeit der erfassten Daten nach vier Stunden vorgegeben, so werden zuvor keine Warnungen ausgegeben, auch wenn die Vorgabe schon zu einem früheren Zeitpunkt, beispielsweise schon eine Stunde vor Ablauf der Zeitspanne von vier Stunden, nicht erfüllt worden ist.

Ist der Wachbogen, der von der jeweiligen Klinik verwendet wird, in dem System zur Überwachung des medizinischen Zustandes einer Vielzahl von Patienten hinterlegt, so erfolgt die Warnung durch Farbkodierung der jeweiligen Bereiche des auf dem Bildschirm dargestellten Wachbogens, in denen sich die erwartete Angabe, für die keine Daten erfasst worden sind, befinden. Dabei werden keine Warnungen für die einzelnen Felder ausgegeben, in denen eine erwartete Angabe ohne erfasste Daten geblieben ist. In den Bereichen sind erwartete Angaben nach vorgegebenen Kriterien zusammengefasst. Derartige Bereiche sind beispielsweise "Perfusion", "Infusion" und "Dekubitus", die jeweils mehrere erwartete Daten umfassen.

Es kann vorgesehen sein, dass durch Auswahl eines solchen Bereiches auf dem Bildschirm ein Dokumentationsblatt aufgerufen werden kann, indem dann die erwartete Angabe, beispielsweise durch Farbkodierung, gekennzeichnet ist, für die keine Daten erfasst worden sind.

Das System zur Überwachung des medizinischen Zustandes einer Vielzahl von Patienten mittels des Verfahrens der vorliegenden Erfindung umfasst einen Prozessor, einen Speicher, eine Eingabevorrichtung und eine Anzeigeeinrichtung. Dabei kann vorgesehen sein, dass
- die Eingabevorrichtung dem Anwender die Angabe der getroffenen medizinischen Maßnahmen für jeden Patienten ermöglicht;
- in dem Speicher die kontinuierlich erfassten Werte von Parametern der ersten und zweiten Gruppe der Vielzahl von Patienten und die getroffenen medizinischen Maßnahmen für jeden dieser Patienten gespeichert sind;
- der Prozessor die Notwendigkeit bestimmt, ob Werte von Parametern der zweiten Gruppe bestimmt werden müssen (Schritt (c)), und, falls vorgesehen, die getroffene medizinische Maßnahme einer vorgebenden Kategorie zuordnet und die Kategorie einem der erfassten Parameter der ersten Gruppe, der für das Vorliegen einer Gesundheitsstörung charakteristisch ist, zuordnet und
- die Anzeigevorrichtung die Kategorie und ausgewählten Parameter gemeinsam und zeitabhängig darstellt.

Ferner können in dem Speicher Datenbanken für die Kategorien und vorgegebenen Merkmale zur Bildung der Kategorien hinterlegt sein.

Das System kann ferner eine Einrichtung zum Überprüfen der Vollständigkeit der Daten umfassen.

Das System kann ein computerimplementiertes System sein.

Nachfolgend wird die Erfindung anhand von Zeichnungen näher erläutet. Dabei zeigen
- Fig. 1: eine schematische Darstellung einer ersten Ausführungsform des erfindungsgemäßen Verfahrens;
- Fig. 2: eine schematische Darstellung eines Bildschirmbildes der erfindungsgemäßen Anzeigevorrichtung;
- Fig. 3: eine weitere schematische Darstellung eines Bildschirmbildes der erfindungsgemäßen Anzeigevorrichtung;
- Fig. 4: eine schematische Darstellung einer zweiten Ausführungsform des erfindungsgemäßen Verfahrens;
- Fig. 5: eine schematische Darstellung eines Bildschirmbildes der erfindungsgemäßen Anzeigevorrichtung, und
- Fig. 6: eine weitere schematische Darstellung eines Bildschirmbildes der erfindungsgemäßen Anzeigevorrichtung.

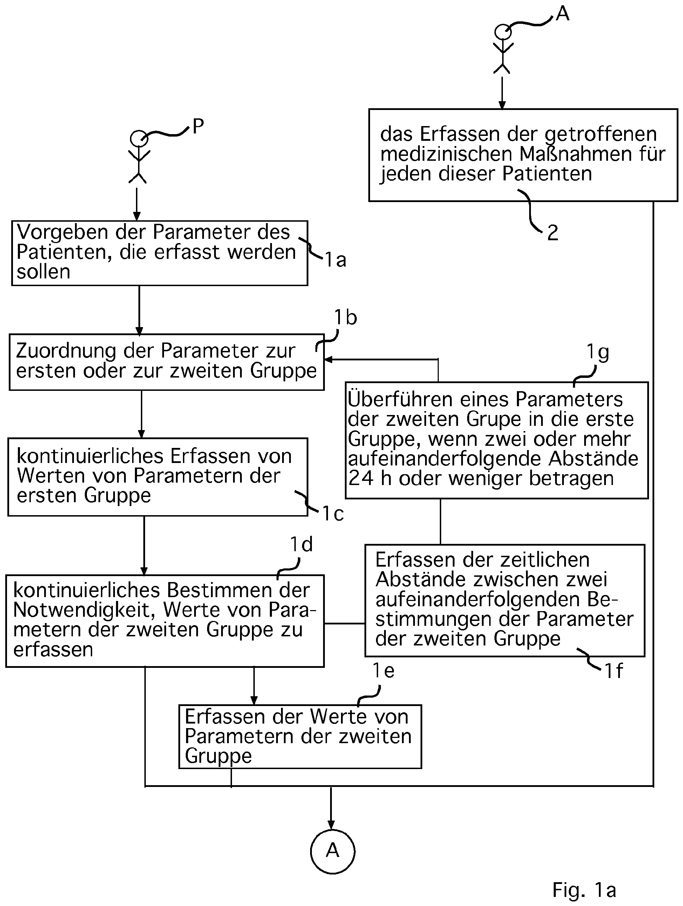

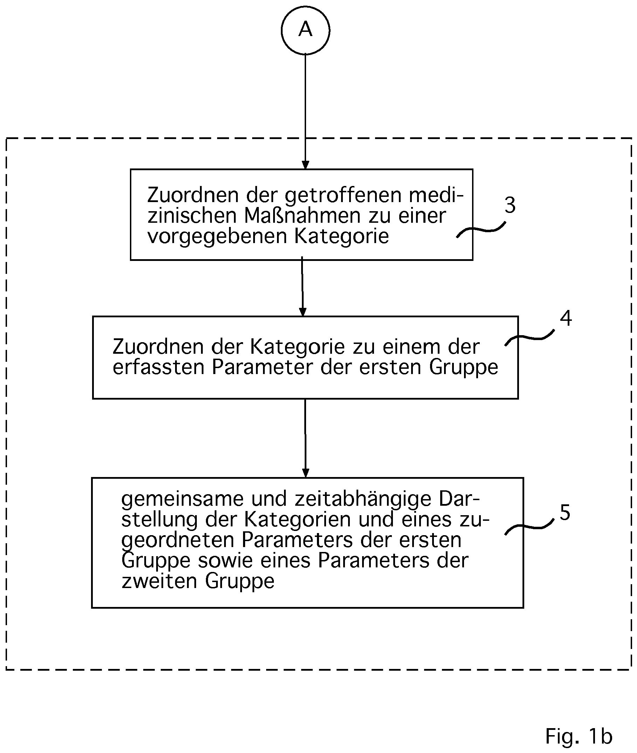

Nach Fig. 1 werden in einer ersten Ausführungsform der Erfindung die physiologischen und/oder pathologischen Parameter des Patienten P, die erfasst werden sollen, bestimmt (1a). Dabei werden die Parameter der ersten Gruppe oder der zweiten Gruppe zugeordnet (1b). Diese Zuordnung kann der Behandler treffen. Alternativ kann die Zuordnung im System bereits hinterlegt sein. Für die Parameter der ersten Gruppe werden kontinuierlich Werte erfasst (1c). Gleichzeitig wird kontinuierlich die Notwendigkeit überprüft, ob Werte von Parametern der zweiten Gruppe bestimmt werden müssen (1d). Diese Überprüfung erfolgt auf Basis von zuvor erfassten Werten von Parametern der ersten Gruppe oder auf Basis von zuvor erfassten Werten von Parametern der ersten Gruppe und der zweiten Gruppe. Immer dann, wenn eine solche Notwendigkeit festgestellt wird, werden Werte von Parametern der zweiten Gruppe erfasst (1e). Gleichzeitig werden die zeitlichen Abstände zwischen den aufeinanderfolgender Erfassungen der Parameter der zweiten Gruppe bestimmt (1f). Liegen zwei oder mehr aufeinanderfolgende Abstände bei einem Parameter der zweiten Gruppe bei 24 h oder weniger, so wird dieser Parameter automatisch von der zweiten Gruppe in die erste Gruppe eingeordnet (1g). Die notwendige Anzahl solcher aufeinander folgender Abstände von 24 h oder weniger, kann vom System oder vom Behandler vorgegeben werden.

Während der so vorgenommenen Erfassung von Werten von Parametern, kann der Behandler A, beispielsweise ein Arzt oder ein Pfleger, nun eine medizinische Maßnahme im Hinblick auf den Zustand des Patienten P treffen, der sich in den erfassten Parametern der ersten oder zweiten Gruppe widerspiegelt. Dabei kann es sich um die Verordnung eines Medikamentes handeln.

Die medizinische Maßnahme, die für einen bestimmten Patienten durch den Behandler getroffen worden ist, wird durch das erfindungsgemäße System, beispielsweise über eine Eingabevorrichtung erfasst 2. Dabei werden der Beginn der getroffenen medizinischen Maßnahme, das vorgesehene Ende der getroffenen medizinischen Maßnahme, der Name des verordneten Medikaments, die Darreichungsform, die Dosis, die Häufigkeit der Dosis pro Tag und die zeitliche Verteilung der Dosierung über den Tag und die Veränderung der Dosierung über einen vorgegebenen Zeitraum erfasst. Ferner wird der Behandler die medizinische Maßnahme zumindest einem der erfassten Parameter der ersten Gruppe, der mit der medizinischen Maßnahme beeinflusst werden soll, zuordnen.

Zu einem späteren Zeitraum eintretende Veränderungen der Maßnahmen sowie deren tatsächliches Ende werden im Lauf der Zeit ebenfalls erfasst.

Die getroffene medizinische Maßnahme wird dann einer vorgegebenen Kategorie zugeordnet 3. Die Kategorie wird dann einem der erfassten Parameter der ersten Gruppe zugeordnet, in der Regel dem Parameter, der mit der getroffenen medizinische Maßnahme beeinflusst werden soll.

Schließlich werden die Kategorie und die ausgewählten Parameter der ersten und zweiten Gruppe gemeinsam und zeitabhängig auf der Anzeigeinrichtung des erfindungsgemäßen Systems dargestellt 5.

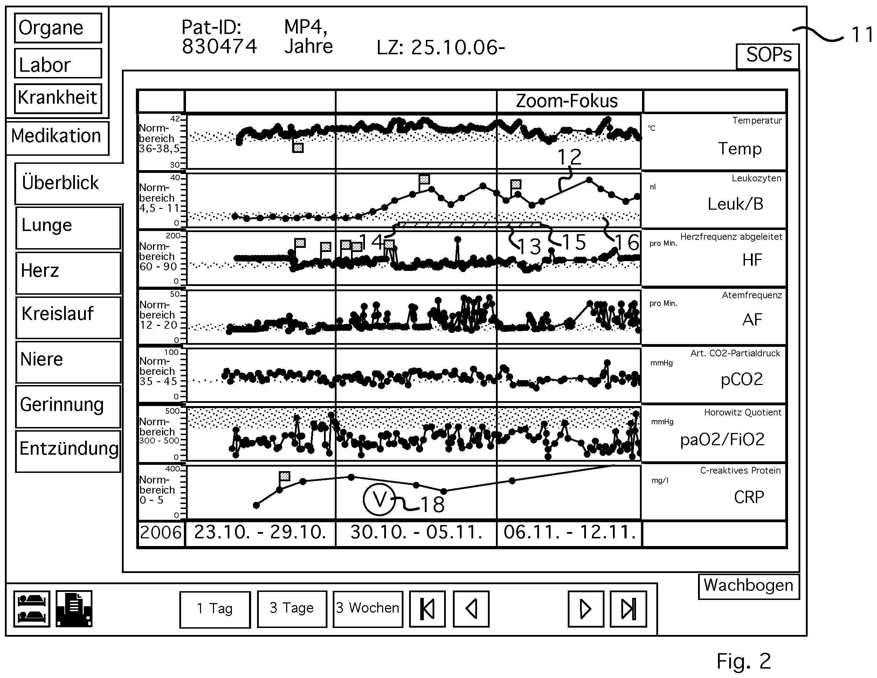

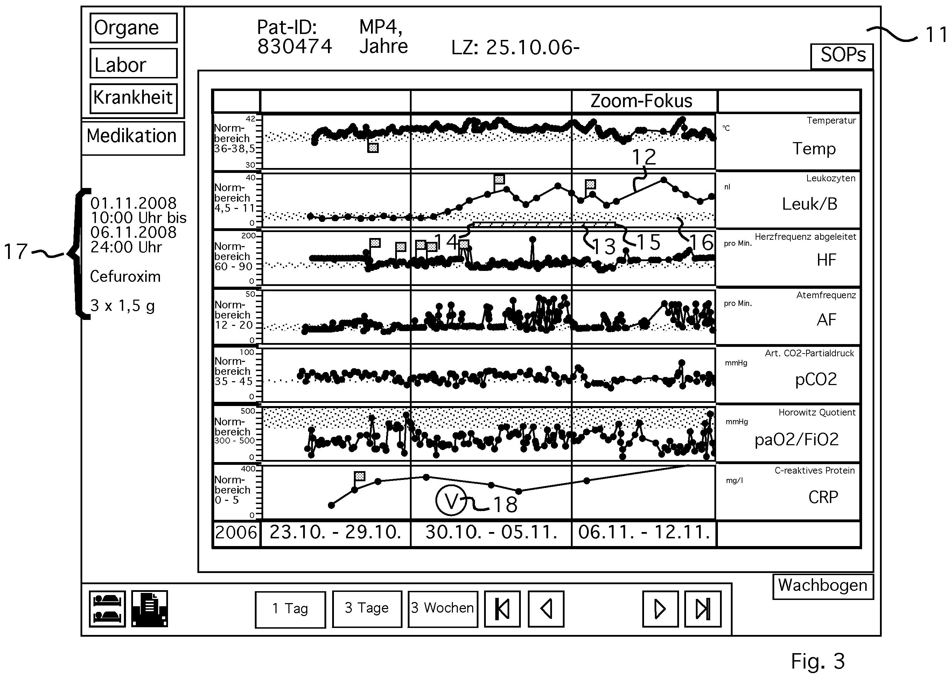

Bildschirmbilder 11 der Anzeigeeinrichtung sind in den Figuren 2 und 3 gezeigt. Dort ist ein Teil der erfassten Parameter gezeigt, nämlich von Parametern der ersten Gruppe und von Parametern der zweiten Gruppe. Die dargestellten Parameter der ersten Gruppe sind die Körpertemperatur (Temp [°C]) des Patienten, die Herzfrequenz (HF [min⁻¹]), die Atemfrequenz (AF [min⁻¹]), der arterielle Kohlendioxid-Partialdruck (pCO2 [mmHg] und der Horowitz-Quotient (paCO2/FiO2 [mmHg]). Die Parameter der zweiten Gruppe sind die Leukozytenzahl (Leuk/B [nl]), die Konzentration des C-reaktiven Proteins (CRP [mg/l]) sowie die Feststellung von Meningokokken.

Der Parameter 12, d. h. die Leukozytenzahl, ist graphisch in Abhängigkeit von der Zeit, die die Abszisse bildet, dargestellt. Der physiologische Parameter 12 ist der zugeordnete Parameter (Schritt d) im Sinne der vorliegenden Erfindung. Dieser physiologische Parameter ist mit einer getroffenen medizinischen Maßnahme verknüpft, wie durch den Balken 13 gezeigt ist. Der Balken 13 lässt in Bezug auf die Abszisse den Anfang 14 und das Ende 15 der getroffenen medizinischen Maßnahme erkennen. Die medizinische Maßnahme ist einer bestimmten Kategorie zugeordnet, wie sich aus der Schraffur des Balkens 13, die für eine bestimmte farbliche Kodierung steht, erkennen lässt. Ferner ist ein weiterer Balken 16 gezeigt, der den erwünschten Bereich des physiologischen Parameters 12 kennzeichnet.

Ferner ist die Feststellung einer viralen Infektion durch ein Symbol 13 gekennzeichnet, dass den Behandler auf das Auffinden von Meningokokken in einer Probe des Patienten hinweist.

Durch die gemeinsame und zeitabhängige Darstellung von Kategorie und ausgewählten Parametern kann ein Behandler ohne Weiteres erkennen, dass eine medizinische Maßnahme getroffen wurde. Anhand der kodierten Darstellung der Kategorie kann er ferner erkennen, welche Art von medizinischer Maßnahme getroffen worden ist. Er kann somit die Werte der ausgewählten Parameter unmittelbar mit der getroffenen medizinischen Maßnahme verknüpfen, was einen erheblichen Vorteil im Hinblick auf den Behandlungserfolg und damit die Behandlungsdauer mit sich bringt.

Sollte der Behandler weitere Informationen in Bezug auf die getroffene medizinische Maßnahme wünschen, so kann er sich diese durch eine Auswahl des Balkens mittels der Eingabevorrichtung anzeigen lassen, wie in Fig. 3 , Bezugszeichen 17, gezeigt. Er kann dann erkennen, dass der Wirkstoff "Cefuroxim" verordnet worden ist, und zwar vom 1. Nov. 2008 (Beginn der medizinischen Maßnahme) bis zum 6. Nov. 2008 (Ende der medizinischen Maßnahme) mit Dosis von 1,5 g, dreimal täglich.

Der Wirkstoff "Cefuroxim", der beispielsweise unter dem Namen "Zinacef" kommerziell vertrieben wird, ist dabei der Kategorie "β-Lactam-Antibiotika" zugeordnet worden. Das Ziel der medizinischen Maßnahme bestand darin, eine bakterielle Entzündung zu bekämpfen. Der Behandler erkennt aus dem Diagramm für die Leukozytenzahl als zugeordnetem physiologischen Parameter 12, dass die Verordnung des Wirkstoffs "Cefuroxim" folgende Wirkung hatte: Die Entzündung wurde zeitweise erfolgreich bekämpft, was sich am fallenden Messwert widerspiegelt.

Durch Auswahl des Symbols 18 erhält der Behandler Detailinformationen zu der festgestellten Meningokokken-Infektion, beispielsweise den Typ der Meningokokken, deren Zahl und gegebenenfalls von festgestellten Resistenzen des Patienten.

Selbstverständlich können weitere getroffene medizinische Maßnahmen und diesen ausgewählten Parameter gleichzeitig mit der in Fig. 2 und Fig. 3 gezeigten medizinischen Maßnahme und deren ausgewählte Parameter angezeigt werden.

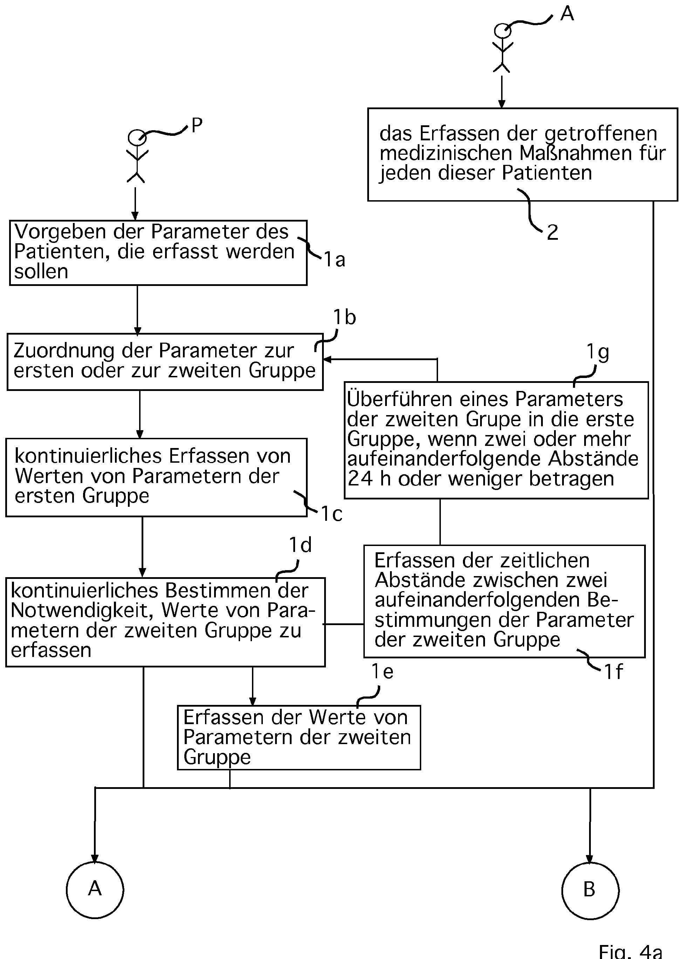

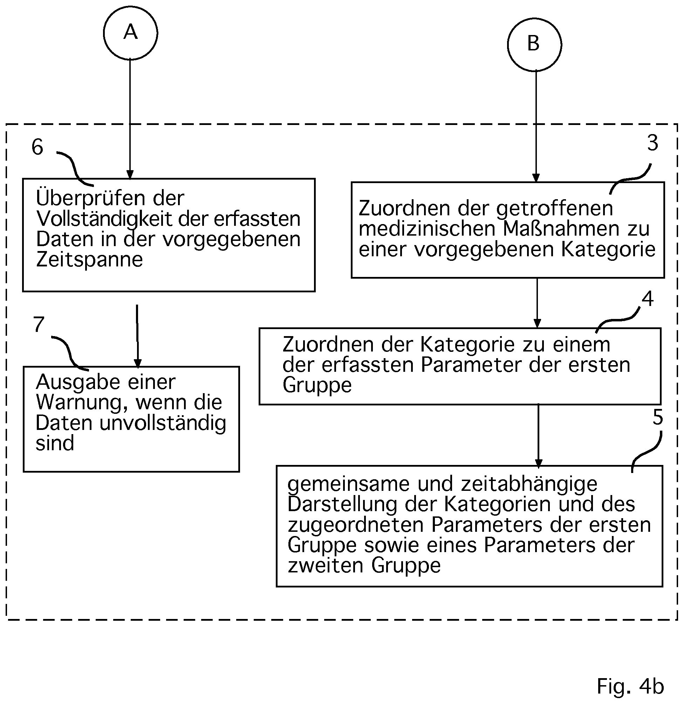

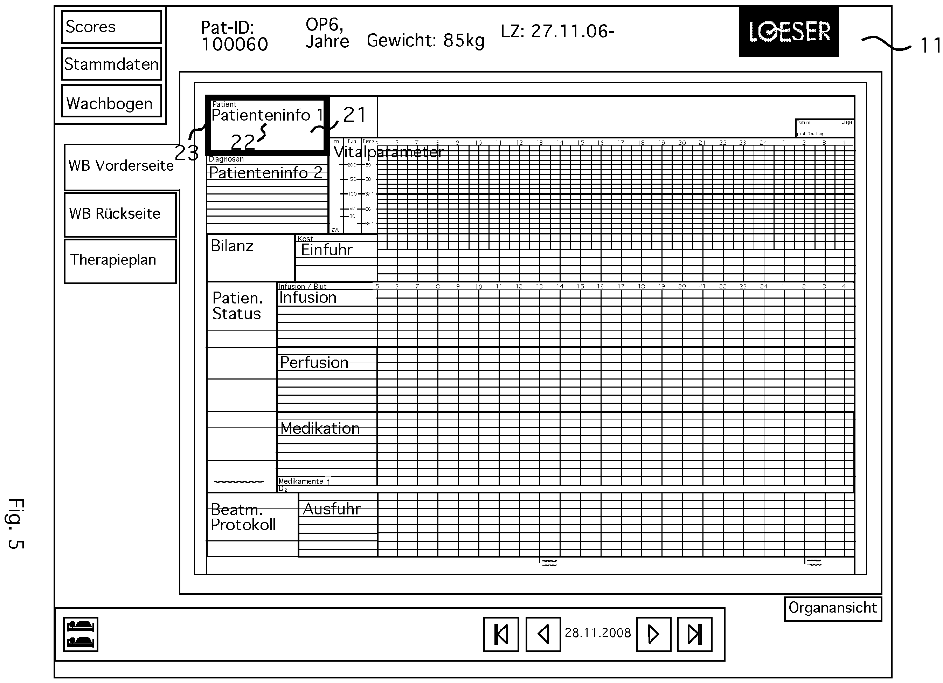

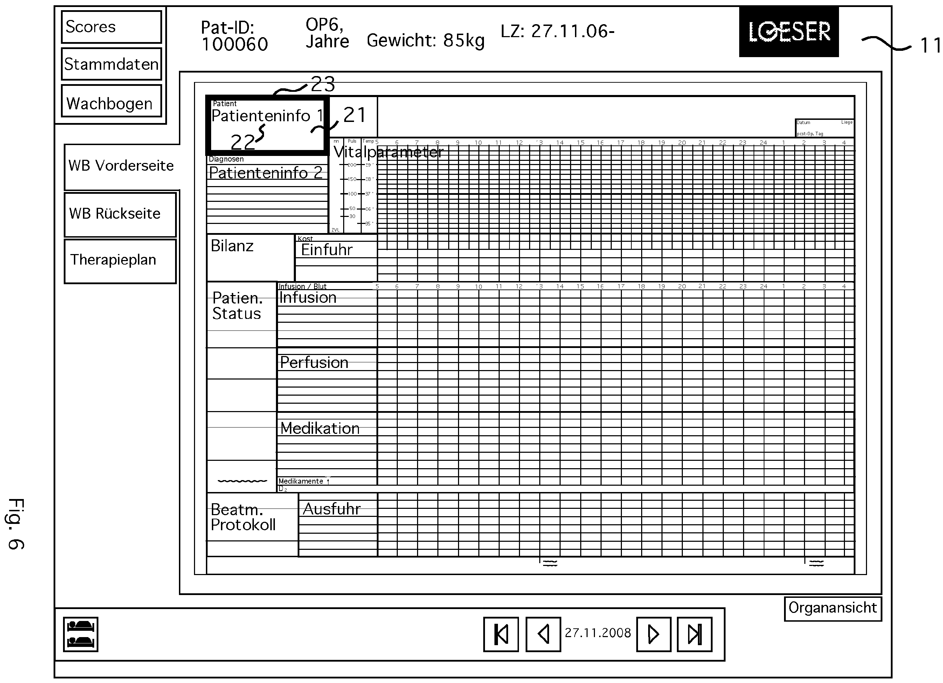

In Fig. 4 ist eine zweite Ausführungsform des erfindungsgemäßen Verfahrens dargestellt, die der ersten Ausführungsform (siehe Fig. 1) entspricht, außer dass zusätzlich eine Überprüfung der erfassten Daten auf Vollständigkeit durchgeführt wird 6. Erweisen sich die erfassten Daten dabei als unvollständig, so wird eine Warnung ausgegeben 7. In Fig. 5 und 6 ist ein Bildschirmbild 11 gezeigt, dass einen Wachbogen wiedergibt. Die Felder des Wachbogens sind in Bereiche 21 eingeteilt, in denen mehrere erwartete Angaben zusammengefasst sind. Die Bereiche sind mit einer Beschriftung 22 und einer Farbkodierung versehen. Ergibt die Überprüfung nach der vorgegebenen Zeitspanne, dass die erfassten Daten unvollständig sind, so ändert sich die Farbe der Beschriftung 22 des Bereiches 21 und die Farbe der Umrandung 23 des Bereiches 21. In Fig. 5 ist die Bildschirmdarstellung 11 vor der Überprüfung 6 gezeigt, während in Fig. 6 die Bildschirmdarstellung 11 nach der Überprüfung 6 gezeigt ist. In Fig. 5 weisen die Bereiche 21 jeweils eine schwarze Beschriftung 22 und eine schwarze Umrandung 23 auf. Es ist in Fig. 6 zu erkennen, dass Warnungen ausgegeben worden sind 7, die durch die jeweils weiße Umrandung 23 der betroffenen Bereiche 21 dargestellt ist. Alternativ oder zusätzlich wäre auch eine weiße Beschriftung 22 der betroffenen Bereiche 21 möglich.

Das Bildschirmbild 11 entspricht dabei - abgesehen von der Beschriftung und Farbkodierung der Bereiche 21 - dem Wachbogen, den das Krankenhaus üblicherweise verwendet.

### Bezugszeichenliste

- P: Patient
- A: Arzt
- 1a: Vorgabe der Parameter des Patienten P, die erfasst werden sollen
- 1b: Zuordnung der vorgegebenen Parameter zur ersten Gruppe oder zur zweiten Gruppe
- 1c: kontinuierliches Erfassen von Werten von Parametern der ersten Gruppe
- 1d: kontinuierliches Bestimmen der Notwendigkeit zur Erfassung von Werten von Parametern der zweiten Gruppe
- 1e: diskontinuierliche Erfassung von Werten von Parametern der zweiten Gruppe
- 1f: Erfassen der zeitlichen Abstände zwischen Erfassungen
- 1g: Überführen eines Parameters der zweiten Gruppe in die erste Gruppe
- 2: Erfassen medizinischer Maßnahmen
- 3: Zuordnen der medizinischen Maßnahmen zu einer Kategorie
- 4: Zuordnen der Kategorie zu einem erfassten physiologischen Parameter
- 5: Darstellen von Kategorie und zugeordnetem Parameter

- 11: Bildschirmbild einer Anzeigevorrichtung
- 12: zugeordneter physiologischer Parameter
- 13: getroffene medizinische Maßnahme
- 14: Beginn der medizinischen Maßnahme
- 15: Ende der medizinischen Maßnahme
- 16: erwünschter Bereich des zugeordneten Parameters der ersten Gruppe
- 17: Detailangaben zur getroffenen medizinischen Maßnahme
- 18: Kennzeichnung der Feststellung eines Virus

- 21: Bereich
- 22: Beschriftung des Bereiches
- 23: Umrandung des Bereiches

## Patentansprüche

1. Verfahren zur Überwachung des medizinischen Zustandes eines Patienten mittels eines computerimplementierten Systems, das einen Prozessor, einen Speicher, eine Eingabevorrichtung und eine Anzeigevorrichtung aufweist, umfassend
(a) die Vorgabe, durch einen Arzt mittels der Eingabevorrichtung, einer ersten Gruppe von physiologischen und/oder pathologischen Parametern des Patienten, die für das Vorliegen einer Gesundheitsstörung charakteristisch sind, wobei die Parameter der ersten Gruppe kontinuierlich bestimmt werden sollen, und die Vorgabe einer zweiten Gruppe von physiologischen und/oder pathologischen Parametern des Patienten, die für das Vorliegen der Gesundheitsstörung charakteristisch sind, wobei die Parameter der zweiten Gruppe diskontinuierlich bestimmt werden sollen;
(b) das kontinuierliche Erfassen von Werten von Parametern der ersten Gruppe, wobei die kontinuierlich erfassten Werte in dem Speicher gespeichert werden;
(c) das kontinuierliche Bestimmen der Notwendigkeit, Werte von Parametern der zweiten Gruppe zu bestimmen, auf Basis von zuvor erfassten Werten von Parametern der ersten Gruppe oder auf Basis von zuvor erfassten Werten von Parametern der ersten Gruppe und der zweiten Gruppe mittels des Prozessors; und
(d) das Erfassen von Werten von Parametern der zweiten Gruppe, wenn in Schritt (c) die Notwendigkeit der Erfassung von Werten von Parametern der zweiten Gruppe festgestellt wurde, zu jedem Zeitpunkt, an dem eine solche Notwendigkeit festgestellt wurde, wobei die Werte in dem Speicher gespeichert werden;
wobei
- ein Parameter kontinuierlich erfasst wird, wenn er ununterbrochen oder in Abständen von 24 h oder weniger bestimmt wird, und ein Parameter diskontinuierlich erfasst wird, wenn er in regelmäßigen oder unregelmäßigen Abständen von mehr als 24 h oder nur dann bestimmt wird, wenn sich dies in Schritt (c) ergibt;
- zur Bestimmung der Notwendigkeit, Parameter der zweiten Gruppe zu bestimmen (Schritt (c)), für zwei oder mehr Parameter der ersten Gruppe jeweils Auslösewerte oder Auslösewertebereiche vorgegebenen sind, bei deren Erreichen zumindest ein Wert zumindest eines Parameters der zweiten Gruppe bestimmt werden muss; und
- die zeitlichen Abstände zwischen zwei aufeinanderfolgenden Bestimmungen von Parametern, die der zweiten Gruppe zugeordnet sind, erfasst werden und ein Parameter aus der zweiten Gruppe in die erste Gruppe überführt wird, wenn eine vorgegebene Anzahl von aufeinanderfolgenden zeitlichen Abständen, die geringer als eine vorgegebene Zeiteinheit sind, erreicht wird, wobei die vorgegebene Anzahl eine Ganzzahl größer gleich zwei ist und die vorgegebene Zeiteinheit 24 Stunden oder weniger ist;
**dadurch gekennzeichnet, dass** die Bestimmung der Notwendigkeit, Parameter der zweiten Gruppe zu bestimmen (Schritt (c))
(c1) das Auswählen von zumindest drei Parametern aus der ersten oder zweiten Gruppe, wobei zumindest einer der drei Parameter ein Parameter der ersten Gruppe ist,
(c2) das Bilden eines mehrdimensionalen Merkmalsraums, wobei jeder der ausgewählten Parameter eine Dimension des Merkmalsraums bildet und die Zeit eine weitere Dimension des Merkmalsraums bildet;
(c3) das Bestimmen von zumindest einem ersten Raumbereich und von zumindest einem zweiten Raumbereich innerhalb des Merkmalsraums, wobei
im ersten Raumbereich jeder der ausgewählten Parameter und/oder die Zeit Werte aufweist, die als Zielwerte vorgegeben worden sind;
im zweiten Raumbereich zumindest einer der Parameter und/oder die Zeit Werte aufweist, die als unerwünschte Werte vorgegeben worden sind;
(c4) das kontinuierliche Einordnen der erfassten Werte für die in Schritt (c1) ausgewählten Parameter, in den Merkmalsraum, wodurch Messpunkte in dem Merkmalsraum gebildet werden; und
(c5) das Feststellen der Notwendigkeit, Parameter der zweiten Gruppe zu erfassen, sobald ein Messpunkt im zweiten Raumbereich liegt;
umfasst.

2. Verfahren nach Anspruch 1, **dadurch gekennzeichnet, dass** die zweite Gruppe der Parameter mikrobiologische Parameter und/oder labormedizinische Parameter umfasst, die für das Vorliegen einer Infektion charakteristisch sind.

3. Verfahren nach Anspruch 1 oder Anspruch 2, **dadurch gekennzeichnet, dass** die zweite Gruppe von Parametern Parameter umfasst, die mittels Blutanalysen gewonnen werden.

4. Verfahren nach Anspruch 1 oder Anspruch 2, **dadurch gekennzeichnet, dass** die zweite Gruppe von Parametern die Ergebnisse bakteriologischer, virologischer und mykologischer Untersuchungen umfasst.

5. Verfahren nach einem der vorstehenden Ansprüche, **dadurch gekennzeichnet, dass** es ferner
(e) das Erfassen der getroffenen medizinischen Maßnahmen für den Patienten, wobei für jede getroffene medizinische Maßnahme zumindest der zeitliche Beginn der medizinischen Maßnahme erfasst wird und die medizinische Maßnahme zumindest einem der erfassten Parameter der ersten Gruppe, der mit der medizinischen Maßnahme beeinflusst werden soll, zugeordnet wird;
(f) das Zuordnen der getroffenen medizinischen Maßnahme zu einer vorgegebenen Kategorie, wobei jede Kategorie eine Vielzahl von potentiellen medizinischen Maßnahmen umfasst;
(g) das Zuordnen der Kategorie, der die medizinische Maßnahme in Schritt (c) zugeordnet worden ist, zu einem der erfassten physiologischen Parameter der ersten Gruppe; und
(h) die gemeinsame und zeitabhängige Darstellung (i) der Kategorie, (ii) des zugeordneten Parameters der ersten Gruppe und (iii) zumindest eines Parameters der zweiten Gruppe;
umfasst.

6. Verfahren nach Anspruch 5, **dadurch gekennzeichnet, dass** die gemeinsame und zeitabhängige Darstellung in Schritt (h) die Darstellung von zumindest zwei Parametern der zweiten Gruppe umfasst, wobei einer der beiden Parameter ein mikrobiologischer Parameter und der andere der beiden Parameter ein labormedizinischer Parameter ist.

7. Verfahren nach einem der vorstehenden Ansprüche, **dadurch gekennzeichnet, dass** ein optischer oder/und akustischer Alarm mittels der Anzeigeeinrichtung ausgelöst wird, wenn bei der Erfassung von Werten von Parametern der zweiten Gruppe ein vorgegebener pathogener Keim und/oder eine Resistenz eines festgestellten Keims gegen ein Arzneimittel festgestellt wird, das dem Patienten verordnet werden soll.

8. Verfahren nach einem der Ansprüche 6 oder 7, **dadurch gekennzeichnet, dass** anhand des Namens des vorordneten Arzneimittels die Kategorie der getroffenen medizinischen Maßnahme unter Rückgriff auf eine Datenbank bestimmt wird, in der der Name des Arzneimittels mit zumindest einem der Merkmale Wirkstoff, Wirkstoffgruppe oder beabsichtigte pharmakologische Wirkung verknüpft ist.

9. Verfahren nach einem der Ansprüche 5 bis 8, **dadurch gekennzeichnet, dass** in Schritt (e) zusätzlich zum Beginn der medizinischen Maßnahme das vorgesehene und/oder tatsächliche Ende dieser medizinischen Maßnahme erfasst wird.

10. Verfahren nach einem der vorstehenden Ansprüche 5 bis 9, **dadurch gekennzeichnet, dass** in Schritt (e) die Kategorie, der zugeordnete Parameter der ersten Gruppe und der zumindest eine Parameter der zweiten Gruppe in einem Diagramm dargestellt werden, dessen Abszisse die Zeit bildet, wobei in dem Diagramme Messwerte des zugeordneten physiologischen Parameters und die Kategorie der getroffenen Maßnahme dargestellt werden.

11. Verfahren nach Anspruch 10, **dadurch gekennzeichnet, dass** die Kategorie der getroffenen Maßnahme als farbig kodierter Balken dargestellt wird, wobei die farbige Kodierung mit dem vorgegebenen Merkmal verknüpft ist.

12. Verfahren nach einem der vorstehenden Ansprüche, **dadurch gekennzeichnet, dass** es ferner das Überprüfen der Vollständigkeit der erfassten Daten und die Ausgabe einer Warnung umfasst, wenn die erfassten Daten ganz oder teilweise unvollständig sind.

## Claims

1. A method for monitoring the medical condition of a patient by means of a computer-implemented system comprising a processor, a memory, an input device, and a display device, the method comprising
(a) the specification, by a physician by means of the input device, of a first group of physiological and/or pathological parameters of the patient that are characteristic of the presence of a disturbance of health, wherein the parameters of the first group are to be determined in a continuous manner, and the specification of a second group of physiological and/or pathological parameters of the patient that are characteristic of the presence of a disturbance of health, wherein the parameters of the second group are to be determined in a discontinuous manner;
(b) the continuous recording of values of parameters of the first group, wherein the continuously recorded values are stored in the memory;
(c) the continuous determination of the necessity to determine values of parameters of the second group on the basis of previously recorded values of parameters of the first group or on the basis of previously recorded values of parameters of the first group and the second group by means of the processor; and
(d) the recording of values of parameters of the second group, if in step (c) the necessity of the recording of values of parameters of the second group has been found, at any time at which such a necessity has been found, wherein the values are stored in the memory;
wherein
- a parameter is continuously recorded, if it is determined non-intermittently or in intervals of 24 hrs or less, and a parameter is discontinuously recorded, if it is determined in regular or irregular intervals of more than 24 hrs or only when this arises in step (c);
- for the determination of the necessity for determining parameters of the second group (step (c)) for two or more parameters of the first group there are each given trigger values or ranges of trigger values at which, if achieved, at least one value of at least one parameter of the second group has to be determined; and
- the time intervals between two successive determinations of parameters assigned to the second group are recorded and a parameter of the second group is transferred into the first group, if a given number of successive time intervals that are smaller than a given unit of time is achieved, wherein the given number is an integer greater than or equal to two and the given unit of time is 24 hours or less;
**characterized in that** the determination of the necessity for determining parameters of the second group (step (c)) comprises
(c1) selection of at least three parameters of the first or second group, wherein at least one of the three parameters is a parameter of the first group,
(c2) formation of a multidimensional feature space, wherein each of the chosen parameters forms a dimension of the feature space and the time forms a further dimension of the feature space;
(c3) determination of at least a first space range and at least a second space range within the feature space, wherein
in the first space range each of the chosen parameters and/or the time have values that have been given as target values;
in the second space range at least one of the parameters and/or the time have values that have been given as unwanted values;
(c4) continuous classification of the recorded values for the parameters chosen in step (c1) into the feature space to form measuring points in the feature space; and
(c5) determination of the necessity for recording parameters of the second group as soon as a measuring point is within the second space range.

2. The method according to claim 1, **characterized in that** the second group of the parameters comprises microbiological parameters and/or laboratory-medical parameters characteristic of the presence of an infection.

3. The method according to claim 1 or claim 2, **characterized in that** the second group of parameters comprises parameters obtained by means of blood analyses.

4. The method according to claim 1 or claim 2, **characterized in that** the second group of parameters comprises the results of bacteriological, virological, and mycological tests.

5. The method according to any one of the preceding claims, **characterized in that** it further comprises
(e) recording the taken medical measures for the patient, wherein for each taken medical measure at least the time-related beginning of the medical measure is recorded and the medical measure is assigned to at least one of the recorded parameters of the first group that shall be affected with the medical measure;
(f) assigning the taken medical measure to a given category, wherein each category comprises a large number of potential medical measures;
(g) assigning the category to which the medical measure in step (c) has been assigned to one of the recorded parameters of the first group; and
(h) commonly and time-dependently representing (i) the category, (ii) the assigned parameter of the first group, and (iii) at least one parameter of the second group.

6. The method according to claim 5, **characterized in that** the common and time-dependent representation in step (h) comprises the representation of at least two parameters of the second group, wherein one of the two parameters is a microbiological parameter and the other of the two parameters is a laboratory-medical parameter.

7. The method according to any one the preceding claims, **characterized in that** an optical or/and audible alert is triggered by means of the display device, if in the recording of values of parameters of the second group a given pathogenic germ and/or a resistance of a found germ to a drug that shall be prescribed for the patient is found.

8. The method according to any one of claims 6 or 7, **characterized in that** by means of the name of the prescribed drug the category of the medical measure taken is determined with recourse to a database wherein the name of the drug is linked with at least one feature of active ingredient, group of active ingredients, or intended pharmacological effect.

9. The method according to any of claims 5 to 8, **characterized in that** in step (e) in addition to the beginning of the medical measure the intended and/or actual end of said medical measure is recorded.

10. The method according to any one the preceding claims 5 to 9, **characterized in that** in step (e) the category, the assigned parameter of the first group, and the at least one parameter of the second group are represented in a diagram the abscissa of which forms the time wherein in the diagram there are represented measured values of the assigned physiological parameter and the category of the taken measure.

11. The method according to claim 10, **characterized in that** the category of the taken measure is represented as color-coded bar wherein the color-coding is linked with the given feature.

12. The method according to any one the preceding claims, **characterized in that** it further comprises the examination of the completeness of the recorded data and the output of a warning if the recorded data are entirely or partially incomplete.

## Revendications

1. Procédé pour la surveillance de l'état de santé d'un patient au moyen d'un système mis en oeuvre par ordinateur qui présente un processeur, une mémoire, un dispositif de saisie et un dispositif indicateur, regroupant
(a) l'objectif par un médecin au moyen d'un dispositif de saisie, d'un premier groupe de paramètres physiologiques et/ou pathologiques du patient qui sont caractéristiques pour la présence d'un trouble de santé, tandis que les paramètres du premier groupe doivent être continuellement déterminés, ainsi que l'objectif d'un deuxième groupe de paramètres physiologiques et/ou pathologiques du patient qui sont caractéristiques pour la présence d'un trouble de santé, tandis que les paramètres du deuxième groupe doivent être déterminés de manière discontinue;
(b) la saisie continuelle de valeurs de paramètres du premier groupe tandis que les valeurs saisies en continu seront enregistrés dans la mémoire;
(c) la détermination en continu de la nécessité de déterminer des valeurs de paramètres du deuxième groupe, sur la base des valeurs de paramètres du premier groupe saisies précédemment, ou sur la base des valeurs de paramètres du premier groupe saisies précédemment et du deuxième groupe au moyen du processeur; et
(d) la saisie de valeurs de paramètres du deuxième groupe si dans l'étape (c) la nécessité de saisir des valeurs de paramètres du deuxième groupe est constatée, à chaque moment où une telle nécessité a été constatée, tandis que les valeurs ont été enregistrées dans la mémoire;
tandis que
- un paramètre est saisi en continu s'il est ininterrompu ou déterminé à intervalles de 24 h ou moins, et un paramètre est saisi en discontinu s'il est déterminé à intervalles réguliers ou irréguliers de plus de 24 h ou dès qu'il apparaît dans l'étape (c);
- pour la détermination de la nécessité de déterminer les paramètres du deuxième groupe (étape (c)), pour deux paramètres ou plus du premier groupe, chaque seuil d'intervention ou domaine de seuil d'intervention correspondant est prédéfini, pour lesquels l'atteinte doit être déterminée par au moins une valeur d'au moins un paramètre du deuxième groupe; et
- les intervalles temporels entre deux déterminations successives de paramètres attribuées au deuxième groupe, seront saisis et un paramètre du deuxième groupe sera transféré dans le premier groupe si un nombre prédéfini d'intervalles temporels successifs, inférieurs à l'unité de temps prédéfinie, est atteint, tandis que le nombre prédéfini est un nombre entier supérieur ou égal à deux et l'unité de temps prédéfinie est de 24 heures ou moins;
de ce fait **caractérisé en ce que** la détermination de la nécessité de déterminer les paramètres du deuxième groupe (étape (c)) comprend
(c1) le choix d'au moins trois paramètres du premier ou du deuxième groupe, tandis qu'au moins un des trois paramètres est un paramètre du premier groupe,
(c2) la formation d'un espace de caractérisation où chacun des paramètres choisis forme une dimension de l'espace de caractérisation et le temps forme une autre dimension de l'espace de caractérisation;
(c3) la détermination d'au moins une première zone d'espace et d'au moins une deuxième zone d'espace au sein de l'espace de caractérisation, tandis que
dans la première zone d'espace, chacun présente les paramètres choisis et/ou le temps présente des valeurs prédéfinies en tant que valeurs cibles;
dans la deuxième zone d'espace, au moins un des paramètres et/ou le temps présente des valeurs prédéfinies comme non-désirées;
(C4) le classement continu des valeurs saisies pour les paramètres choisis dans l'étape (c1) où l'espace de caractérisation et donc des points de mesure sont formés dans l'espace de caractérisation; et
(c5) la constatation de la nécessité de saisir les paramètres du deuxième groupe dès qu'un point de mesure est présent dans la deuxième zone d'espace.

2. Procédé selon revendication 1, de ce fait **caractérisé en ce que** le deuxième groupe de paramètres comprend des paramètres microbiologiques et/ou de laboratoire médical qui sont caractéristiques pour la présence d'une infection.

3. Procédé selon revendication 1 ou revendication 2, de ce fait **caractérisé en ce que** le deuxième groupe de paramètres comprend des paramètres qui seront obtenus au moyen d'analyses sanguines.

4. Procédé selon revendication 1 ou revendication 2, de ce fait **caractérisé en ce que** le deuxième groupe de paramètres comprend les résultats des examens bactériologiques, virologiques et mycologiques.

5. Procédé selon l'une des revendications susmentionnées de ce fait **caractérisé en ce qu'**il comprend en outre
e) la saisie des mesures médicales prises pour les patients où, pour chaque mesure médicale rencontrée, au moins le début temporel de la mesure médicale est saisi et la mesure médicale est attribuée au moins à l'un des paramètres saisis du premier groupe qui doit être influencé par la mesure médicale;
(f) l'attribution des mesures médicales prises pour une catégorie prédéfinie où chaque catégorie comprend une multitude de mesures médicales potentielles;
(g) l'attribution de la catégorie à qui la mesure médicale dans l'étape (c) est attribuée, à l'un des paramètres physiologiques saisie du premier groupe; et
(h) la représentation commune et dépendante du temps (i) de la catégorie, (ii) du paramètre attribué du premier groupe et (iii) au moins d'un paramètre du deuxième groupe.

6. Procédé selon revendication 5 de ce fait **caractérisé en ce que** la représentation commune et dépendante du temps dans l'étape (h) comprend la représentation d'au moins deux paramètres du deuxième groupe où l'un des deux paramètres est un paramètre microbiologique et l'autre est un paramètre de laboratoire médical.

7. Procédé selon l'une des revendications susmentionnées de ce fait **caractérisé en ce qu'**une alarme optique ou/et acoustique est déclenchée au moyen d'un dispositif d'affichage si, lors de la saisie des valeurs de paramètres du deuxième groupe, un germe pathogène prédéfini et/ou une résistance à l'un des germes constatés est constaté(e) face à un médicament qui doit être attribué au patient.

8. Procédé selon l'une des revendications 6 ou 7 de ce fait **caractérisé en ce que**, par l'intermédiaire du nom du médicament attribué, la catégorie de mesure médicale prise est déterminée en s'appuyant sur une base de données selon laquelle le nom du médicament est associé à au moins l'une/l'un des caractéristiques, substances actives ou groupes de substance active ou effets pharmacologiques intentionnés.

9. Procédé selon l'une des revendications 5 à 8 de ce fait **caractérisé en ce que** dans l'étape (e), en plus du début de la mesure médicale, la fin prévue et/ou effective de cette mesure médicale est saisie.

10. Procédé selon l'une des revendications susmentionnées 5 à 9 de ce fait **caractérisé en ce que** dans l'étape (e) la catégorie, le paramètre attribué du premier groupe et au moins l'un des paramètres du deuxième groupe sont représentés dans un diagramme dont l'abscisse forme le temps tandis que les valeurs de mesure du paramètre physiologique attribué et les catégories des mesures prises sont représentées dans le diagramme.

11. Procédé selon la revendication 10 de ce fait **caractérisé en ce que** la catégorie des mesures prises est représentée en couleur par des barres codées où le code en couleur est relié à une caractéristique prédéfinie.

12. Procédé selon l'une des revendications susmentionnées de ce fait **caractérisé en ce qu'**il comprend en outre la vérification de l'intégralité des données saisies et l'émission d'un avertissement si les données saisies sont totalement ou partiellement incomplètes.
